# EUROPEAN PATENT APPLICATION

(11) **EP 4 134 979 A1**
(43) Date of publication of application: **15.02.2023**
(21) Application number: 22168203.2
(22) Date of filing: 13.04.2022
(51) Int. Cl.: G16H 50/50, A61B 5/346

(54) **CARDIAC EVENT RATE LIMITER**

(30) Priority: 15.04.2021 US 202117232026
(71) Applicant: Preventice Solutions, Inc., Rochester, MN 55901 (US)
(72) Inventor: MCCLANAHAN, Timothy, Rochester, 55901 (US); MATRAS, Jake, Rochester, 55901 (US); MCROBERTS, Michael, Rochester, 55901 (US)
(74) Representative: Pfenning, Meinig & Partner mbB

(57) **Abstract**

A method includes assigning a first instance of a cardiac event that occurred in a patient during a period of time to a first bucket of a first plurality of buckets based on a first measured heart rate of the patient during the first instance. A first heart rate threshold of the first bucket is less than the first measured heart rate. The method also includes determining whether the first heart rate threshold exceeds heart rate thresholds of all buckets of the plurality of buckets to which an instance of the cardiac event that occurred in the patient during the period of time is assigned, determining whether a number of instances of the cardiac event that occurred in the patient during the period of time exceeds an event threshold, and preventing the first instance of the cardiac event from being communicated for clinical review.

## Description

### BACKGROUND

Portable monitoring devices for collecting biometric data are becoming increasingly common in diagnosing and treating medical conditions in patients. For example, mobile devices can be used to monitor cardiac data in a patient. This cardiac monitoring can empower physicians with valuable information regarding the occurrence and regularity of a variety of heart conditions and irregularities in patients. Cardiac monitoring can be used, for example, to identify abnormal cardiac rhythms, so that critical alerts can be provided to patients, physicians, or other care providers and patients can be treated.

### BRIEF DESCRIPTION OF THE DRAWINGS

So that the manner in which the features of the present disclosure can be understood in detail, a more particular description of the disclosure, briefly summarized above, may be had by reference to embodiments, some of which are illustrated in the appended drawings. It is to be noted, however, that the appended drawings illustrate only exemplary embodiments and are therefore not to be considered limiting of its scope, may admit to other equally effective embodiments.
Figure 1 illustrates an example system.
Figure 2 is a flowchart of an example method in the system of Figure 1.
Figure 3 is a flowchart of an example method in the system of Figure 1.
Figure 4 illustrates an example cardiac event server in the system of Figure 1.
Figure 5 illustrates an example cardiac event server in the system of Figure 1.
To facilitate understanding, identical reference numerals have been used, where possible, to designate identical elements that are common to the figures. It is contemplated that elements and features of one embodiment may be beneficially incorporated in other embodiments without further recitation.

### DESCRIPTION OF EXAMPLE EMBODIMENTS

### OVERVIEW

According to an embodiment, a method includes determining that a first instance of a cardiac event in a patient should be assigned to a first bucket of a first plurality of buckets based on a first measured heart rate of the patient during the first instance of the cardiac event. The first plurality of buckets are assigned a plurality of heart rate thresholds. A first heart rate threshold of the first bucket of the first plurality of buckets is less than the first measured heart rate. The method also includes communicating the first instance of the cardiac event for clinical review and determining that a second instance of the cardiac event in the patient should be assigned to the first bucket of the first plurality of buckets based on a second measured heart rate of the patient during the second instance of the cardiac event. The second instance occurs after the first instance. The method further includes, in response to determining that the second instance should be assigned to the first bucket of the first plurality of buckets, preventing the second instance from being communicated for clinical review. Other embodiments include an apparatus for performing this method.

According to another embodiment, a method includes assigning a first instance of a cardiac event that occurred in a patient during a period of time to a first bucket of a first plurality of buckets based on a first measured heart rate of the patient during the first instance of the cardiac event. The first plurality of buckets are assigned a plurality of heart rate thresholds. A first heart rate threshold of the first bucket is less than the first measured heart rate. The method also includes determining whether the first heart rate threshold exceeds heart rate thresholds of all buckets of the plurality of buckets to which an instance of the cardiac event that occurred in the patient during the period of time is assigned and determining whether a number of instances of the cardiac event that occurred in the patient during the period of time exceeds an event threshold. The method further includes, in response to determining that the number of instances of the cardiac event that occurred in the patient during the period of time exceeds the event threshold and that the first heart rate threshold does not exceed the heart rate thresholds of all buckets of the plurality of buckets to which an instance of the cardiac event that occurred in the patient during the period of time is assigned, preventing the first instance of the cardiac event from being communicated for clinical review.

### EXAMPLE EMBODIMENTS

As part of mobile cardiac monitoring, a remote monitoring device can collect electrocardiogram (ECG) data for a patient, and transmit the ECG data to a remote server or device for classification and processing. This ECG data can be transmitted in strips, representing ECG readings for a particular period of time (e.g., a few seconds, a few minutes, or longer). A remote server or device can evaluate the strip of ECG data to identify abnormal cardiac rhythms, or other issues, and generate events.

In some circumstances, multiple abnormal cardiac rhythms or other issues can be seen in a given strip ECG of data for a patient, or across multiple ECG data strips for the patient. These abnormal cardiac rhythms (or other issues) can signify cardiac events for a patient. A remote server or device evaluating the ECG data can identify the cardiac events and notify a lab for clinical review. Appropriate care may then be administered to the patient based on the clinical review.

Many patients, however, experience numerous cardiac events over a short period of time. When these events are all reported to the lab, the lab may become inundated with data to review. Additionally, much of the data may not result in the patient receiving different or better care than what the patient was already receiving. For example, if a patient experiences the same type of cardiac event of a similar severity several times during a day, not all of these events need to be sent to the lab for ECG review. When all of these events are sent for clinical review, the lab will perform clinically redundant reviews of ECG due to the similarity between cardiac events, which is sub-optimal in terms of overall lab efficiency.

This disclosure describes a server that uses machine learning to identify the cardiac events detected in a patient and to determine whether those events should be communicated for further clinical review. The server quantifies the severity of a cardiac event by assigning it to a bucket for each of one or more characteristics of the cardiac event, such as heart rate, duration, beat count, etc. Thus, a given cardiac event may be assigned several severity scores corresponding to each characteristic or dimension. Additionally, the time at which the cardiac event occurred is considered, such that a quota of events per time period can be specified, for example "three atrial fibrillation events per day." The specified quota or limit is enforced by considering the one or more quantified severity values in addition to the time at which the event occurred. After the number of events specified by the quota have been sent for clinical review, then the server may limit the number of subsequent cardiac events communicated for clinical review. For example, the server may communicate three atrial fibrillation events early in the day. Later in the day, if a subsequent instance of an atrial fibrillation event is assigned the same severity score(s), then the server may withhold the instance from clinical review. In this manner, the amount of clinically redundant data communicated and reviewed is reduced, which improves lab efficiency and may improve the quality of care received by patients in particular embodiments.

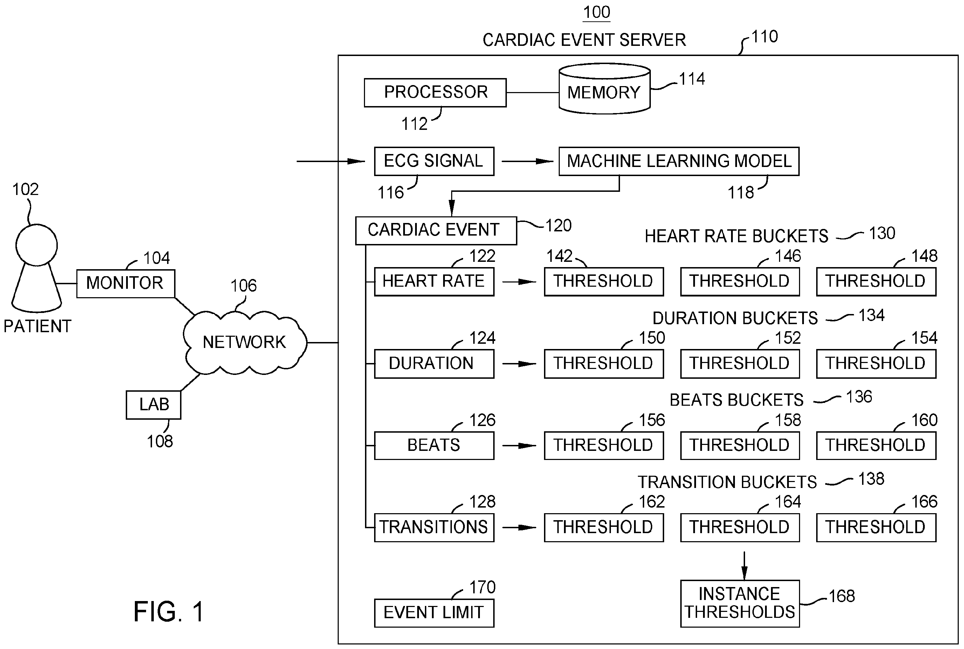

Figure 1 illustrates an example system 100. As seen in Figure 1, the system 100 includes one or more monitors 104, a network 106, a lab 108, and a cardiac event server 110. The cardiac event server 110 applies a machine learning model to information received from the one or more monitors 104 to determine whether certain cardiac events are occurring in patients 102. The cardiac event server 110 quantifies the severity of different instances of cardiac events based on characteristics of the cardiac events such as heart rate, duration, and beat count. The cardiac event server 110 then limits the number of cardiac events of a patient 102 that are communicated to the lab 108 over a period of time. In this manner, the amount of clinically redundant information that the lab 108 reviews is reduced, which improves the health and care of the patients 102 in particular embodiments.

The monitor 104 is a device that couples to a patient 102 to detect cardiac activity of the patient 102. The monitor 104 may attach to the patient 102 using any suitable mechanism. For example, the monitor 104 may include an adhesive that couples the monitor 104 to the body of the patient 102. As another example, the monitor 104 may include a clip that couples the monitor 104 to a casing attached to the patient 102. After the monitor 104 is attached to the patient 102, the monitor 104 may detect cardiac activity within the patient 102. The monitor 104 may produce electric signals that represent the cardiac activity in the patient 102. For example, the monitor 102 may detect the patient's 102 heart beating (e.g., using infrared sensors, electrodes, etc.) and convert the detected heartbeat into electric signals. The monitor 104 communicates the electric signals to the cardiac event server 110 for analysis. For example, the cardiac event server 110 may classify the electric signals as representing regular or irregular cardiac activity. Additionally, the cardiac event server 110 may further classify irregular cardiac activity as particular cardiac events.

The network 106 is any suitable network operable to facilitate communication between the components of the system 100. The network 106 may include any interconnecting system capable of transmitting audio, video, signals, data, messages, or any combination of the preceding. The network 106 may include all or a portion of a public switched telephone network (PSTN), a public or private data network, a local area network (LAN), a metropolitan area network (MAN), a wide area network (WAN), a local, regional, or global communication or computer network, such as the Internet, a wireline or wireless network, an enterprise intranet, or any other suitable communication link, including combinations thereof, operable to facilitate communication between the components.

The lab 108 includes equipment and clinicians that review information from the cardiac event server 110 to determine the appropriate cardiac events to report to the healthcare provider, who uses the information to decide the care to administer to a patient 102. For example, the lab 108 performs clinical review of cardiac events communicated by the cardiac event server 110. Based on the clinical review of the cardiac events, the lab 108 decides which cardiac events and ECG to report to the healthcare provider to preserve or improve the health of the patient 102. If too much redundant information from too many patients 102 is communicated to the lab 108, then the lab 108 may get backed up and review of the information for certain patients 102 may be delayed. As a result, the health of these patients 102 may be jeopardized.

The cardiac event server 110 first applies a machine learning model to identify certain detected cardiac events and quantify their characteristics. The cardiac event server 110 then determines whether to communicate the cardiac events to the lab 108. In this manner, the cardiac event server 110 reduces the amount of clinically redundant information communicated to the lab 108, which improves the health and care of certain patients 102 in particular embodiments. As seen in Figure 1, the cardiac event server 110 includes a processor 112 and a memory 114, which are configured to perform any of the functions or actions of the cardiac event server 110 described herein.

The processor 112 is any electronic circuitry, including, but not limited to microprocessors, application specific integrated circuits (ASIC), application specific instruction set processor (ASIP), and/or state machines, that communicatively couples to memory 114 and controls the operation of the cardiac event server 110. The processor 112 may be 8-bit, 16-bit, 32-bit, 64-bit or of any other suitable architecture. The processor 112 may include an arithmetic logic unit (ALU) for performing arithmetic and logic operations, processor registers that supply operands to the ALU and store the results of ALU operations, and a control unit that fetches instructions from memory and executes them by directing the coordinated operations of the ALU, registers and other components. The processor 112 may include other hardware that operates software to control and process information. The processor 112 executes software stored on the memory 114 to perform any of the functions described herein. The processor 112 controls the operation and administration of the cardiac event server 110 by processing information (e.g., information received from the monitors 104, network 106, and memory 114). The processor 112 may be a programmable logic device, a microcontroller, a microprocessor, any suitable processing device, or any suitable combination of the preceding. The processor 112 is not limited to a single processing device and may encompass multiple processing devices.

The memory 114 may store, either permanently or temporarily, data, operational software, or other information for the processor 112. The memory 114 may include any one or a combination of volatile or non-volatile local or remote devices suitable for storing information. For example, the memory 114 may include random access memory (RAM), read only memory (ROM), magnetic storage devices, optical storage devices, or any other suitable information storage device or a combination of these devices. The software represents any suitable set of instructions, logic, or code embodied in a computer-readable storage medium. For example, the software may be embodied in the memory 114, a disk, a CD, or a flash drive. In particular embodiments, the software may include an application executable by the processor 112 to perform one or more of the functions described herein.

The cardiac event server 110 receives an ECG signal 116 from a monitor 104. The ECG signal 116 represents the cardiac activity of the patient 102. The cardiac event server 110 analyzes the ECG signal 116 to identify and classify the cardiac activity of the patient 102. For example, the cardiac event server 110 may determine whether the cardiac activity is regular or irregular. Additionally, if the cardiac activity is irregular, the cardiac event server 110 may identify or classify a particular cardiac event that is occurring in the patient 102.

The cardiac event server 110 applies a machine learning model 118 (e.g., a deep neural network) to the ECG signal 116 to classify the cardiac activity of the patient 102. For example, the machine learning model 118 may compare the ECG signal 116 to labeled ECG signals to determine which labeled ECG signal the ECG signal 116 most closely resembles. Some of the labeled ECG signals may identify a particular cardiac event. If the cardiac event server 110 or the machine learning model 118 determines that the ECG signal 116 most closely resembles a labeled ECG signal associated with a cardiac event, then the cardiac event server 110 or the machine learning model 118 may determine that the patient 102 is experiencing that cardiac event. Additionally, the machine learning model 118 may measure or determine certain characteristics of the cardiac activity of the patient 102 based on the ECG signal 116. For example, the cardiac event server 110 or the machine learning model 118 may determine a heart rate, a duration, or a beat count of the patient 102 during the cardiac event based on the ECG signal 116.

In certain embodiments, the machine learning model 118 classifies the ECG signal 116 as representing a particular cardiac event 120. The cardiac event 120 may be an irregular cardiac activity that indicates a medical condition of the patient 102. The cardiac event server 110 or the machine learning model 118 may identify any suitable type of cardiac event 120. For example, the cardiac event server 110 or the machine learning model 118 may analyze the ECG signal 116 and detect ventricular tachycardia, supraventricular tachycardia, sinus tachycardia, sinus bradycardia, pause, junctional escape rhythm, idioventricular rhythm, atrial fibrillation with slow ventricular response, atrial fibrillation with rapid ventricular response, atrial fibrillation with controlled ventricular response, third degree heart block, second degree heart block type 2, second degree heart block type 1, 2:1 heart block, first degree heart block with sinus tachycardia, and first degree heart block with sinus bradycardia.

The cardiac event server 110 or the machine learning model 118 may further determine characteristics of the cardiac event 120 (e.g., a heart rate 122 of the patient 102 during the cardia event 120, a duration 124 of the cardiac event 120, a beat count 126 of the patient 102 during the cardiac event 120, or a number of transitions 128 from one type of cardiac event to another type of cardiac event). Each of these characteristics may be measured or determined from the ECG signal 116. For example, the cardiac event server 110 or the machine learning model 118 may count a number of crests or troughs in the ECG signal 116 to determine the heart rate 122 or the beat count 126 of the patient 102 during the cardiac event 120. As another example, the cardiac event server 110 or the machine learning model 118 may measure a time span of the ECG signal 116 to determine a duration 124 of the cardiac event 120. As yet another example, the cardiac event server 110 or the machine learning model 118 may determine a progression or change within the ECG signal 116 over a span of time to determine the number of cardiac rhythm transitions 128.

After the cardiac event server 110 determines the cardiac event 120 and its corresponding characteristics, the cardiac event server 110 assigns the cardiac event 120 to particular buckets based on one or more of the determined characteristics. For example, the cardiac event server 110 may assign the cardiac event 120 to a heart rate bucket 130 based on the heart rate 122. As seen in Figure 1, each heart rate bucket 130 includes a heart rate threshold. For example, the heart rate buckets 130 may apply a threshold 142, a threshold 146, and a threshold 148. The threshold 146 may be greater than the threshold 142, and the threshold 148 may be greater than the threshold 146. The cardiac event server 110 assigns the cardiac event 120 to a particular heart rate bucket 130 depending on whether the heart rate 122 exceeds a particular threshold. For example, if the heart rate 122 exceeds the threshold 142 but not the threshold 146, then the cardiac event server 110 assigns the cardiac event 120 to the heart rate bucket 130 corresponding to the threshold 142. As yet another example, if the heart rate 122 exceeds the threshold 146 but not the threshold 148, then the cardiac event server 110 assigns the cardiac event 120 to the heart rate bucket 130 corresponding to the threshold 146.

The cardiac event server 110 further assigns the cardiac event 120 to other types of buckets based on other characteristics of the cardiac event 120. For example, the cardiac event server 110 may assign the cardiac event 120 to a duration bucket 113 based on the duration 124 of the cardiac event 120. In the example of Figure 1, the cardiac event server 110 may assign the cardiac event 120 to one of three duration buckets 134. A first duration bucket 134 corresponds to a duration threshold 150. A second duration bucket 134 corresponds to a duration threshold 152. A third duration bucket 134 corresponds to a duration threshold 154. The duration threshold 152 exceeds the duration threshold 150. The duration threshold 154 exceeds the duration thresholds 150 and 152. The cardiac event server 110 may assign the cardiac event 120 to a duration bucket 134 based on whether the duration 124 exceeds the threshold corresponding to the duration bucket 134. For example, if the duration 124 exceeds the threshold 150 but not the threshold 152, then the cardiac event server 110 assigns the cardiac event 120 to the duration bucket 134 corresponding to the duration threshold 150. As another example, if the duration 124 exceeds the duration threshold 152 but not the duration threshold 154, then the cardiac event server 110 assigns the cardiac event 120 to the duration bucket 134 corresponding to the duration threshold 152.

As another example, the cardiac event server 110 may assign the cardiac event 120 to a beat bucket 136 based on the beat count 126. In the example of Figure 1, the cardiac event server 110 assigns the cardiac event 120 to one of three beat buckets 136. A first beat bucket 136 corresponds to a beat threshold 156. A second beat bucket 136 corresponds to a beat threshold 158. A third beat bucket 136 corresponds to a beat threshold 160. The cardiac event server 110 may assign the cardiac event 120 to a particular beat bucket 136 depending on whether the beat count 126 exceeds the beat threshold corresponding to the beat bucket 136. For example, if the beat count 126 exceeds the beat threshold 156 but not the beat threshold 158, then the cardiac event server 110 may assign the cardiac event 120 to the beat bucket 136 corresponding to the beat threshold 156. As another example, if the beat count 126 exceeds the beat threshold 158 but not the beat threshold 160, then the cardiac event server 110 may assign the cardiac event 120 to the beat bucket 136 corresponding to the beat threshold 158.

As yet another example, the cardiac event server 110 may assign the cardiac event 120 to a transition bucket 138 based on the number of detected cardiac rhythm transitions 128. In the example of Figure 1, the cardiac event server 110 may assign the cardiac event 120 to one of three transition buckets 138. A first transition bucket 138 corresponds to a transition threshold 162. A second transition bucket 138 corresponds to a transition threshold 164. A third transition bucket 138 corresponds to a transition threshold 166. The cardiac event server 110 may assign the cardiac event 120 to a particular transition bucket 138 based on whether the transitions 128 exceed the transition threshold corresponding to that transition bucket 138. For example, if the number of transitions 128 exceed the transition threshold 164 but not the transition threshold 166, then the cardiac event server 110 assigns the cardiac event 120 to the transition bucket 138 corresponding to the transition threshold 164. As another example, if the number of transitions 128 exceed the transition threshold 166, then the cardiac event server 110 assigns the cardiac event 120 to the transition bucket 138 corresponding to the transition threshold 166.

The cardiac event server 110 uses the thresholds assigned to the buckets to quantify the severity of the cardiac event 120. For example, cardiac events 120 that are assigned to the same heart rate bucket 130 are considered to have the same severity based on their measured heart rates 122. As another example, cardiac events 120 that are assigned to the same duration bucket 134 are considered to have the same severity based on their measured durations 124. Stated differently, the cardiac event server 110 considers cardiac events 120 with similar measured heart rates 122 or similar measured durations 124 to be of the same clinical severity based on heart rate or duration.

In some embodiments, the cardiac event server 110 does not consider the highest threshold of a characteristic to quantify the severity of a cardiac event 120 based on that characteristic. Rather, when a cardiac event 120 is assigned to a bucket with the highest threshold for a characteristic, the cardiac event server 110 considers the measured characteristic for that cardiac event 120 as quantifying the severity of the cardiac event 120 based on that characteristic. For example, if the cardiac event server determines that a cardiac event 120 should be assigned to the heart rate bucket 130 with the heart rate threshold 148, then the cardiac event server 110 does not consider the threshold 148 as quantifying the severity of the cardiac event 120 based on heart rate. Rather, the cardiac event server 110 considers the measured heart rate 122 of the cardiac event 120 as quantifying the severity of the cardiac event 120 based on heart rate.

After the cardiac event server 110 assigns the cardiac event 120 to one or more buckets, the cardiac event server 110 may determine whether to communicate the cardiac event 120 to the lab 108 based on an event limit 170. The event limit 170 represents a limit on the number of cardiac events 120 of a particular type to be communicated to the lab 108 over a period of time (e.g., one day, one hour, one minute, etc.). If the patient 102 has had a number of instances of a particular type of cardiac event exceeding the event limit 170, then the cardiac event server 110 may perform additional analysis to determine whether the cardiac event 120 should be communicated to the lab 108. If the number of instances of the type of cardiac event 120 has not exceeded the event limit 170, then the cardiac event server 110 may communicate the cardiac event 120 to the lab 108 for clinical review.

If the number of instances of a cardiac event 120 that occurred in the patient 102 during the period of time exceeds the event limit 170, the cardiac event server 110 may still communicate the cardiac event 120 to the lab 108 if the severity of the cardiac event 120 is greater than previous instances of the cardiac event 120 that occurred in the patient 102 during the period of time. For example, if the event limit is one and if a first cardiac event 120 is already assigned to the heart rate bucket 130 with the threshold 142, then the cardiac event server 110 may still communicate a second cardiac event 120 to the lab 108 if the second cardiac event 120 is assigned to a heart rate bucket 130 with a threshold that is higher than the threshold 142 (e.g., the heart rate bucket 130 with the threshold 146. As another example, if the first cardiac event 120 were assigned to the heart rate bucket 130 with the threshold 142 and the duration bucket 134 with the threshold 150 and if the second cardiac event 120 were assigned to the heart rate bucket 130 with the threshold 142 and the duration bucket 134 with the threshold 152, the cardiac event server 110 may still communicate the second cardiac event 120 to the lab 108 because the second cardiac event 120 has a more severe duration than the first cardiac event 120. Alternatively, the cardiac event server 110 may prevent the second cardiac event 120 from being communicated to the lab 108 because the second cardiac event 120 does not have a more severe heart rate and a more severe duration than the first cardiac event 120. The rules that the cardiac event server 110 applies to determine whether to communicate a cardiac event 120 to the lab 108 may be configured differently for different types of cardiac events 120.

Generally, the cardiac event server 110 uses the thresholds for the buckets to quantify the severity of various characteristics of a cardiac event 120. However, as discussed above, the cardiac event server 110 may quantify the severity of a characteristic using the measured value of the characteristic rather than the threshold if the cardiac event 120 is assigned to the bucket with the highest threshold. In these instances, the cardiac event server 110 determines whether to communicate a cardiac event 120 to the lab 108 if the measured characteristic for that cardiac event 120 exceeds the measured characteristics of all previous cardiac events 120 during the same period of time (e.g., a day, an hour, a minute, etc.). For example, if a first cardiac event 120 has a first measured heart rate and is assigned to the heart rate bucket 130 with the threshold 148 and if a second cardiac event 120 has a second measured heart rate and is assigned to the heart rate bucket 130 with the threshold 148, then the cardiac event server 110 may communicate the second cardiac event 120 to the lab 108 if the second measured heart rate exceeds the first measured heart rate. In other words, even though the first and second cardiac events 120 are assigned to the same bucket, the cardiac event server 110 may still communicate the second cardiac event 120 because the second measured heart rate indicates a higher severity than the first measured heart rate.

In some embodiments, the cardiac event server 110 may also determine whether the cardiac event 120 should be communicated to the lab 108 based on one or more instance thresholds 168. Each instance threshold 168 may apply to a particular characteristic of the cardiac event 120 (e.g., the heart rate 122, the duration 124, the beat count 126, or the transitions 128). The instance thresholds 168 indicate a limit on the number of cardiac events 120 of a particular type assigned to a particular bucket (e.g., of a same severity) that may be communicated to the lab 108 for clinical review. For example, an instance threshold 168 may set a limit on the number of cardiac events 120 of a particular type assigned to the same heart rate bucket 130 that may be communicated to the lab 108 for clinical review. If the instance threshold 168 for the heart rate buckets 130 is one, then when two cardiac events 120 of the same type are assigned to the same heart rate bucket 130, the cardiac event server 110 may communicate only one of those cardiac events 120 to the lab 108 for clinical review. The cardiac event server 110 may prevent or withhold the other cardiac event 120 from being communicated to the lab 108 for clinical review. In this manner, the amount of information that is sent to the lab 108 for further review is reduced, which allows the lab 108 to review information from other patients 102. In particular embodiments, reducing the amount of information communicated to the lab 108 improves the health of the other patients 102.

In certain embodiments, the cardiac event server 110 assigns the cardiac event 120 to multiple types of buckets based on the characteristics of the cardiac event 120. The cardiac event server 110 then communicates the cardiac event 120 to the lab 108 depending on the number of buckets that exceed their corresponding instance thresholds 168. For example, the cardiac event server 110 may assign the cardiac event 120 to a heart rate bucket 130 based on the heart rate 122 and a duration bucket 134 based on the duration 124. Using the example of Figure 1, the cardiac event server 110 may assign the cardiac event 120 to the heart rate bucket 130 corresponding to the threshold 146 and the duration bucket 134 corresponding to the duration threshold 150. The cardiac event server 110 may then determine whether instance thresholds 168 for the heart rate buckets 130 and the duration buckets 134 have been exceeded. For example, the cardiac event server 110 may determine that the instance threshold 168 for the heart rate bucket 130 corresponding to the heart rate threshold 146 has been exceeded, and the cardiac event server 110 may determine that the instance threshold 168 for the duration bucket 134 corresponding to the duration threshold 150 has not been exceeded. In response, the cardiac event server 110 may communicate the cardiac event 120 to the lab 108 based on the instance threshold 168 for the duration bucket 134 corresponding to the duration threshold 150 not being exceeded. Alternatively, the cardiac event server 110 may prevent or withhold the cardiac event 120 from being communicated to the lab 108 based on the instance threshold 168 for the heart rate bucket 130 corresponding to the heart rate threshold 146 being exceeded.

In particular embodiments, the cardiac event server 110 sets or adjusts the event limit 170 based on one or more factors. For example, the cardiac event server 110 may set or adjust the event limit 170 based on the health of the patient 102. If the health of the patient 102 is deteriorating, the cardiac event server 110 may increase the event limit 170 so that additional cardiac events 120 are communicated to the lab 108 for clinical review. If the health of the patient 102 is improving, then the cardiac event server 110 may decrease the event limit 170 to reduce the number of cardiac events 120 of the patient 102 communicated to the lab 108 for clinical review. As another example, the cardiac event server 110 may set or adjust the event limit 170 based on an age of the patient 102. If a patient 102 is young, then the patent 102 is likely to be in better health. In response, the cardiac event server 110 may reduce the event limit 170 so that fewer cardiac events 120 of the patient 102 are reported to the lab 108. If a patient 102 is elderly, then the patient 102 is likely to be in bad health or likely to present more health risks. In response, the cardiac event server 110 may increase the event limit 170 so that more cardiac events 120 of the patient 102 are reported to the lab 108.

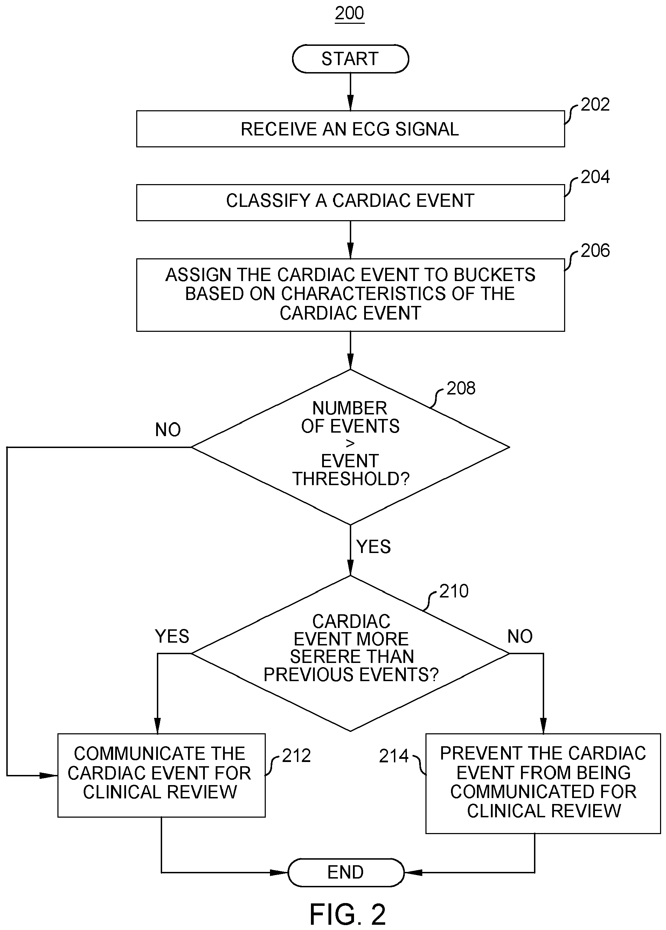

Figure 2 is a flowchart of an example method 200 in the system 100 of Figure 1. The cardiac event server 110 performs the method 200. In particular embodiments, by performing the method 200, the cardiac event server 110 reduces the number of redundant cardiac events 120 that are reported for clinical review, which improves the health and care of patients 102.

The cardiac event server 110 begins by receiving an ECG signal 116 in block 202. The ECG signal 116 is an electric signal that represents cardiac activity of a patient 102. In block 204, the cardiac event server 110 classifies a cardiac event 120 based on the ECG signal 116. The cardiac event server 110 may apply a machine learning model 118 to the ECG signal 116 to classify the cardiac event 120. For example, the cardiac event server 110 may apply the machine learning model 118 to determine whether the ECG signal 116 shows regular or irregular activity. If the ECG signal 116 shows irregular cardiac activity, the machine learning model 118 may then compare the ECG signal 116 to labeled ECG signals to see which labeled ECG signal most closely resembles the ECG signal 116. The labeled ECG signal that most closely resembles the ECG signal 116 may be labeled with a particular cardiac event 120. The machine learning model 118 may classify the ECG signal 116 as indicating the cardiac event 120, thus indicating that the cardiac event 120 is occurring or has occurred in the patient 102.

In block 206, the cardiac event server 110 assigns the cardiac event 120 to buckets based on characteristics of the cardiac event 120. For example, the cardiac event server 110 may assign the cardiac event 120 to one or more heart rate buckets 130, duration buckets 134, beat buckets 136, and transition buckets 138 based on characteristics of the cardiac event 120 such as for example, the heart rate 122, a duration 124, a beat count 126, and a number of transitions 128.

The cardiac event server 110 may assign the cardiac event 120 to particular buckets based on thresholds assigned to those buckets. For example, the cardiac event server 110 may determine a heart rate 122 of the patient 102 when the cardiac event 120 was occurring in the patient 102. The cardiac event server 110 may assign the cardiac event 120 to a particular heart rate bucket 130 based on the heart rate 122 exceeding a heart rate threshold of a particular heart rate bucket 130. As another example, the cardiac event server 110 may determine a duration 124 of the cardiac event 120. The cardiac event server 110 may then assign the cardiac event 120 to a duration bucket 134 based on the duration 124 exceeding a duration threshold of a particular duration bucket 134. The cardiac event server 110 may use these thresholds to quantify the severity of certain characteristics of the cardiac event 120.

In block 208, the cardiac event server 110 determines whether a number of events exceeds an event limit 170. If the number of cardiac events does not exceed the event limit 170, then the cardiac event server 110 communicates the cardiac event 120 for clinical review in block 212. If the number of cardiac events exceeds the event limit 170, the cardiac event server 110 determines in block 210 whether the cardiac event 120 is more severe than previous cardiac events 120 during a period of time (e.g., a day, an hour, a minute, etc.). In other words, the cardiac event server 110 determines whether the cardiac event 120 is more severe than other cardiac events that occurred previously during the period of time. The cardiac event server 110 may use the thresholds of the buckets to which the cardiac event 120 is assigned to determine whether the cardiac event 120 is more severe. For example, if the cardiac event 120 is assigned to a bucket that has a higher threshold than the other buckets to which the previous cardiac events are assigned, then the cardiac event server 110 may determine that the cardiac event 120 is more severe than the previous events. As another example, if the cardiac event 120 is assigned to a bucket with a highest threshold, then the cardiac event server 110 may determine whether the cardiac event 120 has a measured characteristic that is higher than the measured characteristics of the other cardiac events assigned to that bucket. If so, then the cardiac event server 110 may determine that the cardiac event 120 is more severe than the previous events.

If the cardiac event 120 is more severe than previous events, then the cardiac event server 110 communicates the cardiac event 120 for clinical review in block 212. If the cardiac event 120 is not more severe than previous events, then the cardiac event server 110 prevents the cardiac event 120 from being communicated for clinical review in block 214. In this manner, the cardiac event server 110 reduces the number of cardiac events communicated for clinical review by withholding cardiac events that are clinically insignificant from clinical review. As a result, a lab 108 that performs the clinical review is not overloaded with clinically insignificant cardiac events in particular embodiments.

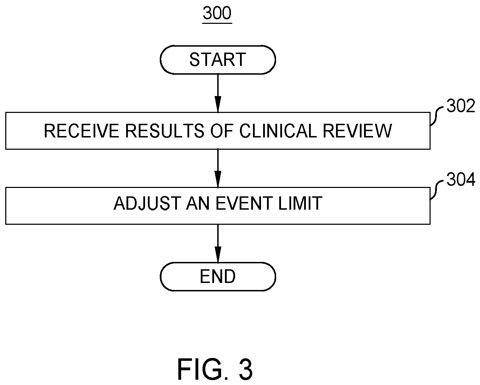

Figure 3 is a flowchart of an example method 300 in the system 100 of Figure 1. The cardiac event server 110 performs the method 300. In particular embodiments, by performing the method 300, the cardiac event server 110 adjusts the number of cardiac events that are communicated for clinical review based on one or more factors.

In block 302, the cardiac event server 110 receives results of a clinical review of a patient 102. The clinical review may indicate whether the health of the patient 102 is improving or deteriorating. In response, the cardiac event server 110 may trigger subsequent reporting and notifications to be sent to the healthcare provider via lab technicians.

In block 304, the cardiac event server 110 adjusts an event limit 170, based on the results of the clinical review. For example, if the clinical review indicates that the health of the patient 102 is improving, then the patient 102 may require fewer clinical reviews. In response, the cardiac event server 110 may reduce the event limit 170, so that fewer cardiac events 120 of the patient 102 are communicated for clinical review. As another example, if the clinical review indicates that the health of the patient 102 is deteriorating, then the patient 102 may require additional clinical review. In response, the cardiac event server 110 increases the event limit 170 so that more cardiac events 120 of the patient 102 are communicated for clinical review. In this manner, the cardiac event server 110 improves the health and care provided to the patient 102 without overloading the lab 108 that performs the clinical reviews, in particular embodiments.

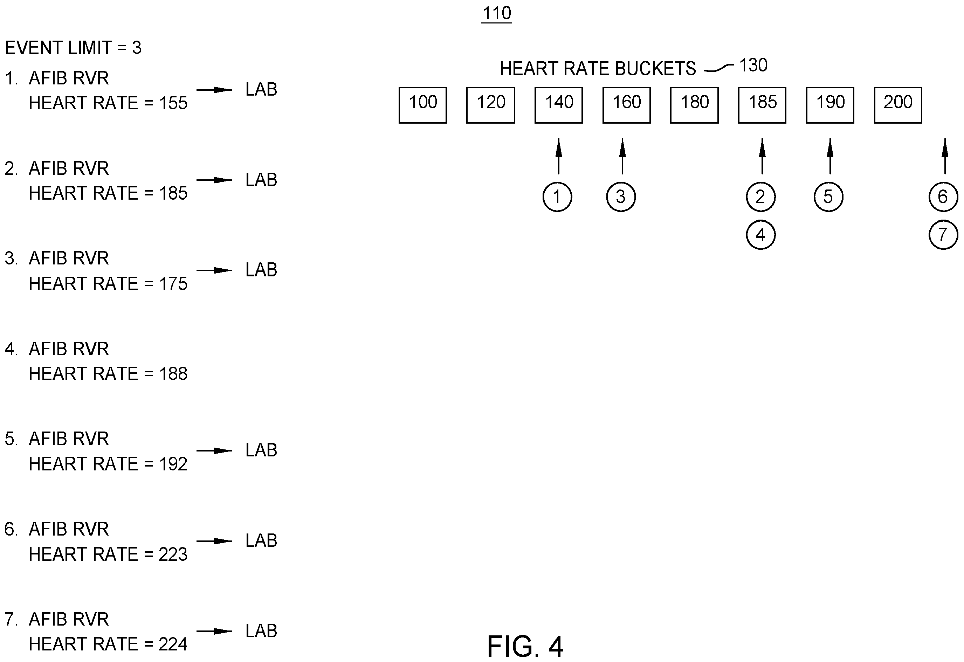

Figure 4 illustrates an example cardiac event server 110 in the system 100 of Figure 1. In the example of Figure 4, the cardiac event server 110 determines the cardiac events 120 of a patient 102 that should be communicated for clinical review.

As seen in Figure 4, the cardiac event server 110 implements an event limit of three and an instance threshold of one. The cardiac event server 110 receives ECG signals 116 for a patient 102 that indicate multiple instances of atrial fibrillation with rapid ventricular response (AFib RVR). The cardiac event server 110 detects a first instance of AFib RVR with a heart rate of 155. The cardiac event server 110 assigns the first instance of AFib RVR to a heart rate bucket 130 corresponding to a heart rate threshold of 140. Additionally, the cardiac event server 110 communicates the first instance of AFib RVR to the lab 108 for clinical review, because the event limit of three has not been exceeded.

The cardiac event server 110 detects a second instance of AFib RVR with a measured heart rate of 185. The cardiac event server 110 assigns the second instance of AFib RVR to a heart rate bucket 130 corresponding to a heart rate threshold of 185. The cardiac event server 110 also communicates the second instance of AFib RVR to the lab 108 for clinical review, because the event limit of three has not yet been exceeded.

The cardiac event server 110 detects a third instance of AFib RVR with a measured heart rate of 175. The cardiac event server 110 assigns the third instance of AFib RVR to a heart rate bucket 130 corresponding to a heart rate threshold of 160. The cardiac event server 110 also communicates the third instance of AFib RVR to the lab 108 for clinical review, because the event limit of three has not yet been exceeded.

The cardiac event server 110 detects a fourth instance of AFib RVR with a measured heart rate of 188. The cardiac event server 110 assigns the fourth instance of AFib RVR to the heart rate bucket 130 corresponding to the heart rate of 185. Both the second instance of AFib RVR and the fourth instance of AFib RVR are assigned to that heart rate bucket 130. The cardiac event server 110 does not communicate the fourth instance of AFib RVR to the lab 108, because the event limit of three has been exceeded and because the fourth instance of AFib RVR is not more severe than previous instances of AFib RVR.

The cardiac event server 110 detects a fifth instance of AFib RVR with a measured heart rate of 192. The cardiac event server 110 assigns the fifth instance of AFib RVR to a heart rate bucket 130 corresponding to the heart rate threshold 190. The cardiac event server 110 communicates the fifth instance of AFib RVR to the lab 108 for clinical review even though the event limit of three has been exceeded, because the heart rate threshold of 190 is higher than the heart rate thresholds of the heart rate buckets 130 to which the previous instances of AFib RVR were assigned (e.g., heart rate thresholds of 140, 160, and 185).

The cardiac event server 110 detects a sixth instance of AFib RVR with a measured heart rate of 223. The cardiac event server 110 assigns the sixth instance of AFib RVR to a heart rate bucket 130 corresponding to the heart rate threshold 200. The cardiac event server 110 communicates the sixth instance of AFib RVR to the lab 108 for clinical review even though the event limit of three has been exceeded, because no other instances of AFib RVR have been assigned to the heart rate bucket 130 corresponding to the heart rate threshold of 200.

The cardiac event server 110 detects a seventh instance of AFib RVR with a measure heart rate of 224. The cardiac event server 110 assigns the seventh instance of AFib RVR to the heart rate bucket 130 corresponding to the heart rate threshold of 200. The cardiac event server 110 communicates the seventh instance of AFib RVR to the lab 108 for clinical review even though the event limit of three has been exceeded, because the measured heart rate of 224 for the seventh instance of AFib RVR is higher than the other measured heart rate (e.g., 223) of the sixth instance of AFib RVR assigned to the heart rate bucket 130 corresponding to the heart rate threshold of 200.

In this manner, the cardiac event server 110 determines the instances of AFib RVR that are clinically significant and communicates those instances to the lab 108 for clinical review. Additionally, the cardiac event server 110 will consider instances of AFib RVR that are more severe than previous instances as clinically significant, because these instances indicate that the patient's 102 health is deteriorating.

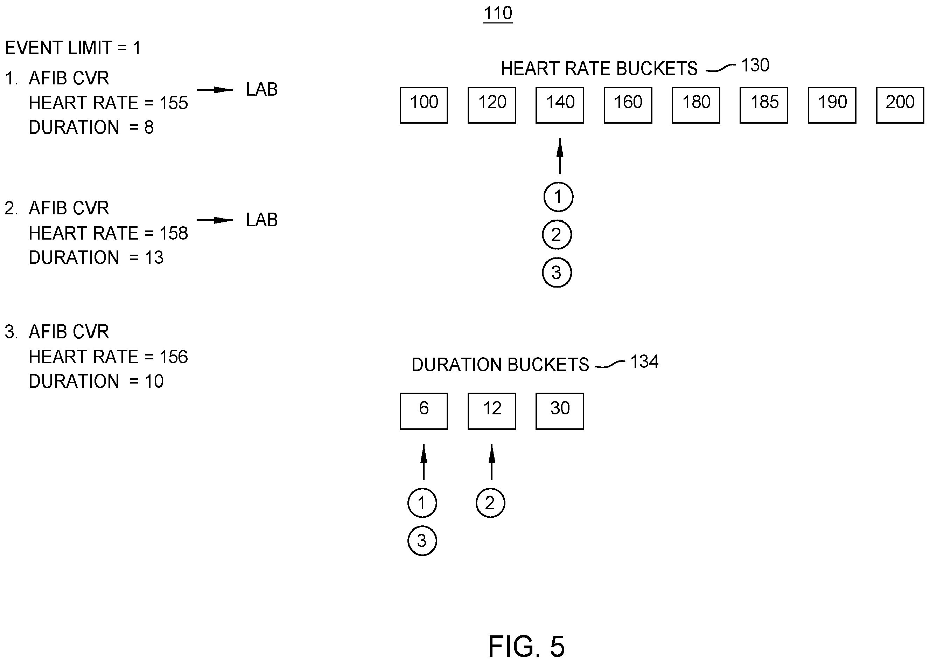

Figure 5 illustrates an example cardiac event server 110 in the system 100 of Figure 1. In the example of Figure 5, the cardiac event server 110 determines which instances of atrial fibrillation with controlled ventricular rate (AFib CVR) are clinically significant and should be communicated to a lab 108 for clinical review.

As seen in Figure 5, the cardiac event server 110 implements an event limit of one for AFib CVR 130. The cardiac event server 110 detects a first instance of AFib CVR with a heart rate of 155 and a duration of 8. The cardiac event server 110 assigns the first instance of AFib CVR to a heart rate bucket 130 corresponding to a heart rate threshold of 140 and a duration bucket 134 corresponding to a duration threshold of 6. The cardiac event server 110 also communicates the first instance of AFib CVR to the lab 108 for clinical review, because the event limit of one has not yet been exceeded.

The cardiac event server 110 detects a second instance of AFib CVR with a heart rate of 158 and a duration of 13. The cardiac event server 110 assigns the second instance of AFib CVR to the heart rate bucket 130 corresponding to the heart rate threshold 140 and the duration bucket 134 corresponding to a duration threshold of 12. The cardiac event server 110 communicates the second instance of AFib CVR to the lab 108 for clinical review even though the event limit of one has been exceeded, because the duration threshold (e.g., 12) of the duration bucket 134 to which the second instance of AFib CVR is assigned is higher than the duration threshold (e.g., 6) of the duration bucket 134 to which the first instance of AFib CVR is assigned..

The cardiac event server 110 detects a third instance of AFib CVR with a heart rate of 156 and a duration of ten. The cardiac event server 110 assigns the third instance of AFib CVR to the heart rate bucket 130 corresponding to the heart rate threshold of 140 and the duration bucket 134 corresponding to the duration threshold of six. The cardiac event server 110 prevents or withholds the third instance of AFib CVR from being communicated to the lab 108 for clinical review because the event limit of one has been exceeded and because the third instance of AFib CVR is not more severe than the first or second instances of AFib CVR. Notably, the third instance of AFiB CVR is assigned to the same heart rate bucket 130 and the same duration bucket 134 as the first instance of AFib CVR. In this manner, the cardiac event server 110 determines which instances of AFib CVR are clinically significant and communicates those instances to the lab 108 for clinical review.

In summary, a cardiac event server 110 uses machine learning to identify cardiac events 120 in a patient 102 and to determine whether those cardiac events 120 should be communicated for further clinical review. The cardiac event server 110 assigns cardiac events 120 to one or more buckets depending on certain characteristics of the cardiac event 120, such as heart rate 122, duration 124, and beat count 126. The cardiac event server 110 may then limit the number of cardiac events 120 communicated for clinical review over a certain period of time. For example, the cardiac event server 110 may communicate only one cardiac event 120 per day. If two separate instances of a cardiac event 120 are assigned to the same bucket, then the cardiac event server 110 withholds one of the instances from clinical review. In this manner, the amount of clinically redundant data communicated and reviewed is reduced, which improves the health and care of other patients 102 in particular embodiments.

In the preceding, reference is made to embodiments presented in this disclosure. However, the scope of the present disclosure is not limited to specific described embodiments. Instead, any combination of the described features and elements, whether related to different embodiments or not, is contemplated to implement and practice contemplated embodiments. Furthermore, although embodiments disclosed herein may achieve advantages over other possible solutions or over the prior art, whether or not a particular advantage is achieved by a given embodiment is not limiting of the scope of the present disclosure. Thus, the preceding aspects, features, embodiments and advantages are merely illustrative and are not considered elements or limitations of the appended claims except where explicitly recited in a claim(s).

As will be appreciated by one skilled in the art, the embodiments disclosed herein may be embodied as a system, method or computer program product. Accordingly, aspects may take the form of an entirely hardware embodiment, an entirely software embodiment (including firmware, resident software, micro-code, *etc*.) or an embodiment combining software and hardware aspects that may all generally be referred to herein as a "circuit," "module" or "system." Furthermore, aspects may take the form of a computer program product embodied in one or more computer readable medium(s) having computer readable program code embodied thereon.

Any combination of one or more computer readable medium(s) may be utilized. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium is any tangible medium that can contain, or store a program for use by or in connection with an instruction execution system, apparatus or device.

Program code embodied on a computer readable medium may be transmitted using any appropriate medium, including but not limited to wireless, wireline, optical fiber cable, RF, *etc.,* or any suitable combination of the foregoing.

Computer program code for carrying out operations for aspects of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present disclosure are described below with reference to flowchart illustrations and/or block diagrams of methods, apparatus (systems) and computer program products according to embodiments presented in this disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the Figures illustrate the architecture, functionality and operation of possible implementations of systems, methods and computer program products according to various embodiments. In this regard, each block in the flowchart or block diagrams may represent a module, segment or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The present subject-matter includes, inter alia, the following aspects:
1. A method comprising:
   determining that a first instance of a cardiac event in a patient should be assigned to a first bucket of a first plurality of buckets based on a first measured heart rate of the patient during the first instance of the cardiac event, wherein the first plurality of buckets are assigned a plurality of heart rate thresholds, wherein a first heart rate threshold of the first bucket of the first plurality of buckets is less than the first measured heart rate;
   communicating the first instance of the cardiac event for clinical review;
   determining that a second instance of the cardiac event in the patient should be assigned to the first bucket of the first plurality of buckets based on a second measured heart rate of the patient during the second instance of the cardiac event, wherein the second instance occurs after the first instance; and
   in response to determining that the second instance should be assigned to the first bucket of the first plurality of buckets, preventing the second instance from being communicated for clinical review.
2. The method of aspect 1, wherein communicating the first instance of the cardiac event for clinical review is in response to determining that, at a time of the first instance of the cardiac event, an event limit exceeds a number of instances of the cardiac event in the patient communicated for clinical review in a same day as the first instance of the cardiac event.
3. The method of aspect 2, further comprising:
   determining that, at a time of a third instance of the cardiac event in the patient, the number of instances of the cardiac event exceeds the event limit, wherein the third instance occurs after the second instance;
   determining that the third instance of the cardiac event should be assigned to a second bucket of the first plurality of buckets based on a third measured heart rate of the patient during the third instance of the cardiac event, wherein a second heart rate threshold of the second bucket of the first plurality of buckets is higher than the first heart rate threshold; and
   in response to determining that the third instance of the cardiac event should be assigned to the second bucket of the first plurality of buckets, communicating the third instance of the cardiac event for clinical review even though the number of instances of the cardiac event exceeds the event limit.
4. The method of aspect 2, further comprising:
   determining that the first instance of the cardiac event should be assigned to a first bucket of a second plurality of buckets based on a duration of the first instance of the cardiac event, wherein the second plurality of buckets are assigned a plurality of duration thresholds, wherein a first duration threshold of the first bucket of the second plurality of buckets is less than the duration of the first instance of the cardiac event;
   receiving an indication of a third instance of the cardiac event in the patient and a third measured heart rate of the patient during the third instance of the cardiac event;
   determining that, at a time of the third instance of the cardiac event, the number of instances of the cardiac event exceeds the event limit;
   determining that the third instance of the cardiac event should be assigned to the first bucket of the first plurality of buckets based on the third measured heart rate;
   determining that the third instance of the cardiac event should be assigned to a second bucket of the second plurality of buckets based on a duration of the third instance of the cardiac event, wherein a second duration threshold of the second bucket of the second plurality of buckets is less than the duration of the third instance of the cardiac event, wherein the first duration threshold is less than the second duration threshold; and
   in response to determining that the third instance of the cardiac event should be assigned to the second bucket of the second plurality of buckets, communicating the third instance of the cardiac event for clinical review even though the number of instances of the cardiac event exceeds the event limit and even though the third instance of the cardiac event is assigned to the first bucket of the first plurality of buckets.
5. The method of aspect 2, further comprising adjusting the event limit.
6. The method of aspect 2, further comprising:
   determining that a third measured heart rate of the patient during a third instance of the cardiac event and a fourth measured heart rate of the patient during a fourth instance of the cardiac event exceed a highest heart rate threshold of the plurality of heart rate thresholds;
   in response to determining that the third measured heart rate exceeds the highest heart rate threshold of the plurality of heart rate thresholds, communicating the third instance of the cardiac event for clinical review; and
   in response to determining that the fourth measured heart rate exceeds the highest heart rate threshold of the plurality of heart rate thresholds and the third measured heart rate, communicating the fourth instance of the cardiac event for clinical review.
7. The method of aspect 1, wherein communicating the first instance of the cardiac event for clinical review is further based on one or more of a heartbeat count during the first instance of the cardiac event or a number of transitions to a different cardiac event during the first instance of the cardiac event.
8. An apparatus comprising:
   a memory; and
   a hardware processor communicatively coupled to the memory, the hardware processor configured to:
      determine that a first instance of a cardiac event in a patient should be assigned to a first bucket of a first plurality of buckets based on a first measured heart rate of the patient during the first instance of the cardiac event, wherein the first plurality of buckets are assigned a plurality of heart rate thresholds, wherein a first heart rate threshold of the first bucket of the first plurality of buckets is less than the first measured heart rate;
      communicate the first instance of the cardiac event for clinical review;
      determine that a second instance of the cardiac event in the patient should be assigned to the first bucket of the first plurality of buckets based on a second measured heart rate of the patient during the second instance of the cardiac event, wherein the second instance occurs after the first instance; and
      in response to determining that the second instance should be assigned to the first bucket of the first plurality of buckets, prevent the second instance from being communicated for clinical review.
9. The apparatus of aspect 8, wherein communicating the first instance of the cardiac event for clinical review is in response to determining that, at a time of the first instance of the cardiac event, an event limit exceeds a number of instances of the cardiac event in the patient communicated for clinical review in a same day as the first instance of the cardiac event.
10. The apparatus of aspect 9, the hardware processor further configured to:
   determine that, at a time of a third instance of the cardiac event in the patient, the number of instances of the cardiac event exceeds the event limit, wherein the third instance occurs after the second instance;
   determine that the third instance of the cardiac event should be assigned to a second bucket of the first plurality of buckets based on a third measured heart rate of the patient during the third instance of the cardiac event, wherein a second heart rate threshold of the second bucket of the first plurality of buckets is higher than the first heart rate threshold; and
   in response to determining that the third instance of the cardiac event should be assigned to the second bucket of the first plurality of buckets, communicate the third instance of the cardiac event for clinical review even though the number of instances of the cardiac event exceeds the event limit.
11. The apparatus of aspect 9, the hardware processor further configured to:
   determine that the first instance of the cardiac event should be assigned to a first bucket of a second plurality of buckets based on a duration of the first instance of the cardiac event, wherein the second plurality of buckets are assigned a plurality of duration thresholds, wherein a first duration threshold of the first bucket of the second plurality of buckets is less than the duration of the first instance of the cardiac event;
   receive an indication of a third instance of the cardiac event in the patient and a third measured heart rate of the patient during the third instance of the cardiac event;
   determine that, at a time of the third instance of the cardiac event, the number of instances of the cardiac event exceeds the event limit;
   determine that the third instance of the cardiac event should be assigned to the first bucket of the first plurality of buckets based on the third measured heart rate;
   determine that the third instance of the cardiac event should be assigned to a second bucket of the second plurality of buckets based on a duration of the third instance of the cardiac event, wherein a second duration threshold of the second bucket of the second plurality of buckets is less than the duration of the third instance of the cardiac event, wherein the first duration threshold is less than the second duration threshold; and
   in response to determining that the third instance of the cardiac event should be assigned to the second bucket of the second plurality of buckets, communicate the third instance of the cardiac event for clinical review even though the number of instances of the cardiac event exceeds the event limit and even though the third instance of the cardiac event is assigned to the first bucket of the first plurality of buckets.
12. The apparatus of aspect 9, the hardware processor further configured to adjust the event limit.
13. The apparatus of aspect 9, the hardware processor further configured to:
   determine that a third measured heart rate of the patient during a third instance of the cardiac event and a fourth measured heart rate of the patient during a fourth instance of the cardiac event exceed a highest heart rate threshold of the plurality of heart rate thresholds;
   in response to determining that the third measured heart rate exceeds the highest heart rate threshold of the plurality of heart rate thresholds, communicate the third instance of the cardiac event for clinical review; and
   in response to determining that the fourth measured heart rate exceeds the highest heart rate threshold of the plurality of heart rate thresholds and the third measured heart rate, communicate the fourth instance of the cardiac event for clinical review.
14. The apparatus of aspect 8, wherein communicating the first instance of the cardiac event for clinical review is further based on one or more of a heartbeat count during the first instance of the cardiac event or a number of transitions to a different cardiac event during the first instance of the cardiac event.
15. A method comprising:
   assigning a first instance of a cardiac event that occurred in a patient during a period of time to a first bucket of a first plurality of buckets based on a first measured heart rate of the patient during the first instance of the cardiac event, wherein the first plurality of buckets are assigned a plurality of heart rate thresholds, wherein a first heart rate threshold of the first bucket is less than the first measured heart rate;
   determining whether the first heart rate threshold exceeds heart rate thresholds of all buckets of the plurality of buckets to which an instance of the cardiac event that occurred in the patient during the period of time is assigned;
   determining whether a number of instances of the cardiac event that occurred in the patient during the period of time exceeds an event threshold; and
   in response to determining that the number of instances of the cardiac event that occurred in the patient during the period of time exceeds the event threshold and that the first heart rate threshold does not exceed the heart rate thresholds of all buckets of the plurality of buckets to which an instance of the cardiac event that occurred in the patient during the period of time is assigned, preventing the first instance of the cardiac event from being communicated for clinical review.
16. The method of aspect 15, wherein the period of time is a same day as the first instance of the cardiac event.
17. The method of aspect 15, further comprising:
   determining that, at a time of a second instance of the cardiac event in the patient, the number of instances of the cardiac event exceeds the event limit, wherein the second instance occurs after the first instance;
   determining that the second instance of the cardiac event should be assigned to a second bucket of the first plurality of buckets based on a second measured heart rate of the patient during the second instance of the cardiac event, wherein a second heart rate threshold of the second bucket of the first plurality of buckets is higher than the first heart rate threshold; and
   in response to determining that the second instance of the cardiac event should be assigned to the second bucket of the first plurality of buckets, communicating the second instance of the cardiac event for clinical review even though the number of instances of the cardiac event exceeds the event limit.
18. The method of aspect 15, further comprising:
   determining that the first instance of the cardiac event should be assigned to a first bucket of a second plurality of buckets based on a duration of the first instance of the cardiac event, wherein the second plurality of buckets are assigned a plurality of duration thresholds, wherein a first duration threshold of the first bucket of the second plurality of buckets is less than the duration of the first instance of the cardiac event;
   receiving an indication of a second instance of the cardiac event in the patient and a second measured heart rate of the patient during the second instance of the cardiac event;
   determining that, at a time of the second instance of the cardiac event, the number of instances of the cardiac event exceeds the event limit;
   determining that the second instance of the cardiac event should be assigned to the first bucket of the first plurality of buckets based on the second measured heart rate;
   determining that the second instance of the cardiac event should be assigned to a second bucket of the second plurality of buckets based on a duration of the second instance of the cardiac event, wherein a second duration threshold of the second bucket of the second plurality of buckets is less than the duration of the second instance of the cardiac event, wherein the first duration threshold is less than the second duration threshold; and
   in response to determining that the second instance of the cardiac event should be assigned to the second bucket of the second plurality of buckets, communicating the second instance of the cardiac event for clinical review even though the number of instances of the cardiac event exceeds the event limit and even though the second instance of the cardiac event is assigned to the first bucket of the first plurality of buckets.
19. The method of aspect 15, further comprising adjusting the event limit.
20. The method of aspect 15, further comprising:
   determining that a second measured heart rate of the patient during a second instance of the cardiac event and a third measured heart rate of the patient during a third instance of the cardiac event exceed a highest heart rate threshold of the plurality of heart rate thresholds;
   in response to determining that the second measured heart rate exceeds the highest heart rate threshold of the plurality of heart rate thresholds, communicating the second instance of the cardiac event for clinical review; and
   in response to determining that the third measured heart rate exceeds the highest heart rate threshold of the plurality of heart rate thresholds and the second measured heart rate, communicating the third instance of the cardiac event for clinical review.

## Claims

1. A method comprising:
determining that a first instance of a cardiac event in a patient should be assigned to a first bucket of a first plurality of buckets based on a first measured heart rate of the patient during the first instance of the cardiac event, wherein the first plurality of buckets are assigned a plurality of heart rate thresholds, wherein a first heart rate threshold of the first bucket of the first plurality of buckets is less than the first measured heart rate;
communicating the first instance of the cardiac event for clinical review;
determining that a second instance of the cardiac event in the patient should be assigned to the first bucket of the first plurality of buckets based on a second measured heart rate of the patient during the second instance of the cardiac event, wherein the second instance occurs after the first instance; and
in response to determining that the second instance should be assigned to the first bucket of the first plurality of buckets, preventing the second instance from being communicated for clinical review.

2. The method of claim 1, wherein communicating the first instance of the cardiac event for clinical review is in response to determining that, at a time of the first instance of the cardiac event, an event limit exceeds a number of instances of the cardiac event in the patient communicated for clinical review in a same day as the first instance of the cardiac event.

3. The method of claim 2, further comprising:
determining that, at a time of a third instance of the cardiac event in the patient, the number of instances of the cardiac event exceeds the event limit, wherein the third instance occurs after the second instance;
determining that the third instance of the cardiac event should be assigned to a second bucket of the first plurality of buckets based on a third measured heart rate of the patient during the third instance of the cardiac event, wherein a second heart rate threshold of the second bucket of the first plurality of buckets is higher than the first heart rate threshold; and
in response to determining that the third instance of the cardiac event should be assigned to the second bucket of the first plurality of buckets, communicating the third instance of the cardiac event for clinical review even though the number of instances of the cardiac event exceeds the event limit.

4. The method of claim 2, further comprising:
determining that the first instance of the cardiac event should be assigned to a first bucket of a second plurality of buckets based on a duration of the first instance of the cardiac event, wherein the second plurality of buckets are assigned a plurality of duration thresholds, wherein a first duration threshold of the first bucket of the second plurality of buckets is less than the duration of the first instance of the cardiac event;
receiving an indication of a third instance of the cardiac event in the patient and a third measured heart rate of the patient during the third instance of the cardiac event;
determining that, at a time of the third instance of the cardiac event, the number of instances of the cardiac event exceeds the event limit;
determining that the third instance of the cardiac event should be assigned to the first bucket of the first plurality of buckets based on the third measured heart rate;
determining that the third instance of the cardiac event should be assigned to a second bucket of the second plurality of buckets based on a duration of the third instance of the cardiac event, wherein a second duration threshold of the second bucket of the second plurality of buckets is less than the duration of the third instance of the cardiac event, wherein the first duration threshold is less than the second duration threshold; and
in response to determining that the third instance of the cardiac event should be assigned to the second bucket of the second plurality of buckets, communicating the third instance of the cardiac event for clinical review even though the number of instances of the cardiac event exceeds the event limit and even though the third instance of the cardiac event is assigned to the first bucket of the first plurality of buckets.

5. The method of claim 2, further comprising adjusting the event limit.

6. The method of claim 2, further comprising:
determining that a third measured heart rate of the patient during a third instance of the cardiac event and a fourth measured heart rate of the patient during a fourth instance of the cardiac event exceed a highest heart rate threshold of the plurality of heart rate thresholds;
in response to determining that the third measured heart rate exceeds the highest heart rate threshold of the plurality of heart rate thresholds, communicating the third instance of the cardiac event for clinical review; and
in response to determining that the fourth measured heart rate exceeds the highest heart rate threshold of the plurality of heart rate thresholds and the third measured heart rate, communicating the fourth instance of the cardiac event for clinical review.

7. The method of claim 1, wherein communicating the first instance of the cardiac event for clinical review is further based on one or more of a heartbeat count during the first instance of the cardiac event or a number of transitions to a different cardiac event during the first instance of the cardiac event.

8. An apparatus comprising:
a memory; and
a hardware processor communicatively coupled to the memory, the hardware processor configured to:
determine that a first instance of a cardiac event in a patient should be assigned to a first bucket of a first plurality of buckets based on a first measured heart rate of the patient during the first instance of the cardiac event, wherein the first plurality of buckets are assigned a plurality of heart rate thresholds, wherein a first heart rate threshold of the first bucket of the first plurality of buckets is less than the first measured heart rate;
communicate the first instance of the cardiac event for clinical review;
determine that a second instance of the cardiac event in the patient should be assigned to the first bucket of the first plurality of buckets based on a second measured heart rate of the patient during the second instance of the cardiac event, wherein the second instance occurs after the first instance; and
in response to determining that the second instance should be assigned to the first bucket of the first plurality of buckets, prevent the second instance from being communicated for clinical review.

9. The apparatus of claim 8, wherein communicating the first instance of the cardiac event for clinical review is in response to determining that, at a time of the first instance of the cardiac event, an event limit exceeds a number of instances of the cardiac event in the patient communicated for clinical review in a same day as the first instance of the cardiac event.

10. The apparatus of claim 9, the hardware processor further configured to:
determine that, at a time of a third instance of the cardiac event in the patient, the number of instances of the cardiac event exceeds the event limit, wherein the third instance occurs after the second instance;
determine that the third instance of the cardiac event should be assigned to a second bucket of the first plurality of buckets based on a third measured heart rate of the patient during the third instance of the cardiac event, wherein a second heart rate threshold of the second bucket of the first plurality of buckets is higher than the first heart rate threshold; and
in response to determining that the third instance of the cardiac event should be assigned to the second bucket of the first plurality of buckets, communicate the third instance of the cardiac event for clinical review even though the number of instances of the cardiac event exceeds the event limit.

11. The apparatus of claim 9, the hardware processor further configured to:
determine that the first instance of the cardiac event should be assigned to a first bucket of a second plurality of buckets based on a duration of the first instance of the cardiac event, wherein the second plurality of buckets are assigned a plurality of duration thresholds, wherein a first duration threshold of the first bucket of the second plurality of buckets is less than the duration of the first instance of the cardiac event;
receive an indication of a third instance of the cardiac event in the patient and a third measured heart rate of the patient during the third instance of the cardiac event;
determine that, at a time of the third instance of the cardiac event, the number of instances of the cardiac event exceeds the event limit;
determine that the third instance of the cardiac event should be assigned to the first bucket of the first plurality of buckets based on the third measured heart rate;
determine that the third instance of the cardiac event should be assigned to a second bucket of the second plurality of buckets based on a duration of the third instance of the cardiac event, wherein a second duration threshold of the second bucket of the second plurality of buckets is less than the duration of the third instance of the cardiac event, wherein the first duration threshold is less than the second duration threshold; and
in response to determining that the third instance of the cardiac event should be assigned to the second bucket of the second plurality of buckets, communicate the third instance of the cardiac event for clinical review even though the number of instances of the cardiac event exceeds the event limit and even though the third instance of the cardiac event is assigned to the first bucket of the first plurality of buckets.

12. The apparatus of claim 9, the hardware processor further configured to adjust the event limit.

13. The apparatus of claim 9, the hardware processor further configured to:
determine that a third measured heart rate of the patient during a third instance of the cardiac event and a fourth measured heart rate of the patient during a fourth instance of the cardiac event exceed a highest heart rate threshold of the plurality of heart rate thresholds;
in response to determining that the third measured heart rate exceeds the highest heart rate threshold of the plurality of heart rate thresholds, communicate the third instance of the cardiac event for clinical review; and
in response to determining that the fourth measured heart rate exceeds the highest heart rate threshold of the plurality of heart rate thresholds and the third measured heart rate, communicate the fourth instance of the cardiac event for clinical review.

14. The apparatus of claim 8, wherein communicating the first instance of the cardiac event for clinical review is further based on one or more of a heartbeat count during the first instance of the cardiac event or a number of transitions to a different cardiac event during the first instance of the cardiac event.

15. A method comprising:
assigning a first instance of a cardiac event that occurred in a patient during a period of time to a first bucket of a first plurality of buckets based on a first measured heart rate of the patient during the first instance of the cardiac event, wherein the first plurality of buckets are assigned a plurality of heart rate thresholds, wherein a first heart rate threshold of the first bucket is less than the first measured heart rate;
determining whether the first heart rate threshold exceeds heart rate thresholds of all buckets of the plurality of buckets to which an instance of the cardiac event that occurred in the patient during the period of time is assigned;
determining whether a number of instances of the cardiac event that occurred in the patient during the period of time exceeds an event threshold; and
in response to determining that the number of instances of the cardiac event that occurred in the patient during the period of time exceeds the event threshold and that the first heart rate threshold does not exceed the heart rate thresholds of all buckets of the plurality of buckets to which an instance of the cardiac event that occurred in the patient during the period of time is assigned, preventing the first instance of the cardiac event from being communicated for clinical review.

16. The method of claim 15, wherein the period of time is a same day as the first instance of the cardiac event.

17. The method of claim 15, further comprising:
determining that, at a time of a second instance of the cardiac event in the patient, the number of instances of the cardiac event exceeds the event limit, wherein the second instance occurs after the first instance;
determining that the second instance of the cardiac event should be assigned to a second bucket of the first plurality of buckets based on a second measured heart rate of the patient during the second instance of the cardiac event, wherein a second heart rate threshold of the second bucket of the first plurality of buckets is higher than the first heart rate threshold; and
in response to determining that the second instance of the cardiac event should be assigned to the second bucket of the first plurality of buckets, communicating the second instance of the cardiac event for clinical review even though the number of instances of the cardiac event exceeds the event limit.

18. The method of claim 15, further comprising:
determining that the first instance of the cardiac event should be assigned to a first bucket of a second plurality of buckets based on a duration of the first instance of the cardiac event, wherein the second plurality of buckets are assigned a plurality of duration thresholds, wherein a first duration threshold of the first bucket of the second plurality of buckets is less than the duration of the first instance of the cardiac event;
receiving an indication of a second instance of the cardiac event in the patient and a second measured heart rate of the patient during the second instance of the cardiac event;
determining that, at a time of the second instance of the cardiac event, the number of instances of the cardiac event exceeds the event limit;
determining that the second instance of the cardiac event should be assigned to the first bucket of the first plurality of buckets based on the second measured heart rate;
determining that the second instance of the cardiac event should be assigned to a second bucket of the second plurality of buckets based on a duration of the second instance of the cardiac event, wherein a second duration threshold of the second bucket of the second plurality of buckets is less than the duration of the second instance of the cardiac event, wherein the first duration threshold is less than the second duration threshold; and
in response to determining that the second instance of the cardiac event should be assigned to the second bucket of the second plurality of buckets, communicating the second instance of the cardiac event for clinical review even though the number of instances of the cardiac event exceeds the event limit and even though the second instance of the cardiac event is assigned to the first bucket of the first plurality of buckets.

19. The method of claim 15, further comprising adjusting the event limit.

20. The method of claim 15, further comprising:
determining that a second measured heart rate of the patient during a second instance of the cardiac event and a third measured heart rate of the patient during a third instance of the cardiac event exceed a highest heart rate threshold of the plurality of heart rate thresholds;
in response to determining that the second measured heart rate exceeds the highest heart rate threshold of the plurality of heart rate thresholds, communicating the second instance of the cardiac event for clinical review; and
in response to determining that the third measured heart rate exceeds the highest heart rate threshold of the plurality of heart rate thresholds and the second measured heart rate, communicating the third instance of the cardiac event for clinical review.
